(19) Europäisches Patentamt
European Patent Office
Office européen des brevets

(11) Veröffentlichungsnummer: **0 425 840 A2**

(12) # EUROPÄISCHE PATENTANMELDUNG

(21) Anmeldenummer: **90119216.1**

(51) Int. Cl.[5]: **A61K 31/53**

(22) Anmeldetag: **06.10.90**

(30) Priorität: **31.10.89 DE 3936283**

(43) Veröffentlichungstag der Anmeldung: **08.05.91 Patentblatt 91/19**

(84) Benannte Vertragsstaaten: **AT BE CH ES FR GB GR IT LI LU NL SE**

(71) Anmelder: **Jaeger, Karl-Heinz, Dr. med.**
**Matthofstrand 9**
**CH-6005 Luzern(CH)**

(72) Erfinder: **Jaeger, Karl-Heinz, Dr. med.**
**Matthofstrand 9**
**CH-6005 Luzern(CH)**

(74) Vertreter: **Siewers, Gescha, Dr.**
**Rechtsanwälte Dres. Harmsen, Utescher pp.**
**Adenauerallee 28**
**W-2000 Hamburg 1(DE)**

(54) **Verwendung von Methenamine zur Behandlung von Viruserkrankungen und von malignen Neoplasien.**

(57) Die Erfindung betrifft die Verwendung von Methenamine zur Behandlung von Viruserkrankungen oder malignen Neoplasien.

EP 0 425 840 A2

Xerox Copy Centre

# VERWENDUNG VON METHENAMINE ZUR BEHANDLUNG VON VIRUSERKRANKUNGEN UND VON MALIGNEN NEOPLASIEN

Die Erfindung betrifft die Verwendung von Methenamine zur Behandlung von Viruserkrankungen und zur Behandlung von malignen Neoplasien.

Unter Viren versteht man nach heutiger Definition infektiöse und potenziell pathogene Nukleoproteineinheiten, die nur einen Typ der Nukleinsäure, entweder RNA oder DNA enthalten und die sich mit Hilfe dieses genetischen Materials in einer Wirtszelle reproduzieren. Viren haben keine biochemische Selbständigkeit; zur Reproduktion bedarf es immer der lebenden Wirtszelle. Eine chemotherapeutische Beeinflussung des Virus ist demnach meist nur über den dazwischen geschalteten Wirtsorganismus möglich.

Als maligne Neoplasien werden heute Erkrankungen zusammengefaßt, die sich durch die biologischen Kriterien des Formverfalls, das heißt, der Polymorphie der Zellen, und der Automonie des Tumors auszeichnen. Die Autonomie des Tumors findet ihren Ausdruck in der anhaltenden Wachstumstendenz, da die Regulierung des Wachstums von den übergeordneten Steuerungseinrichtungen auf die Zelle selbst verlagert wird. Man nimmt heute an, daß die Fehlsteuerung von Veränderungen im Bereich der DNA ausgeht.

Da Viren in der Lage sind, genetisches Materials auf Zellen zu übertragen und da Tumorzellen den phänotypischen Ausdruck für einen veränderten Genotyp der Zelle darstellen, ist schon früh angenommen worden, daß Viren die Ursache von Tumor bildungen sein können. Heute ist mit Sicherheit bekannt, daß bestimmte tierische Tumore und ebenfalls einige eher gutartige menschliche Tumore durch Viren hervorgerufen werden. Es ist bereits bekannt, daß auch einige maligne Neoplasien bei Menschen durch Virusinfektionen verursacht werden, wie beispielsweise bestimmte Formen der Leukämie. In jüngster Zeit gibt es auch Beweise dafür, das nicht nur Tumore beim Menschen, sondern auch eine ganze Reihe von mehr oder weniger chronisch verlaufenden Erkrankungen von sogenannten "slow virus" ausgelöst werden können.

Die Kenntnisse über Beziehungen zwischen Virusinfektionen und malignen Neoplasien haben schon früh Anlaß zu der Vermutung gegeben, daß bestimmte Arzneimittel sowohl gegen Viren als auch gegen Tumore wirksam sein könnten. Experimentell hat sich zeigen lassen, daß es tatsächlich eine Reihe von Verbindungen gibt, die bei beiden Erkrankungen einsetzbar sind, auch wenn kaum eine dieser Verbindungen wegen der beträchtlichen Nebenwirkungen eine breitere Anwendung gefunden hat. Am bekanntesten sind noch die Thyminantimetaboliten, wie 5-Fluor-, 5-Brom- und 5-Joduracil sowie deren Desoxyriboside.

Es besteht daher immer noch ein Bedarf nach Arzneimitteln, die zu einer kausalen Therapie von Virusinfektionen oder malignen Neoplasien geeignet sind.

Überraschenderweise wurde jetzt festgestellt, daß eine schon seit langem bekannte Substanz, nämlich Methenamine, meist als Hexamethylentetramin bezeichnet und als HMT abgekürzt, eine Möglichkeit bietet, direkt in den intrazellulären Stoffwechsel eukaryotischer Zellen einzugreifen und somit zur Behandlung von Virusinfektionen und malignen Neoplasien geeignet ist.

Methenamine entspricht der folgenden chemischen Formel

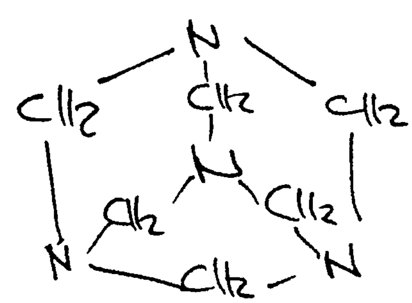

Es handelt sich um ein weißes geruchloses Pulver, das in Wasser sehr leicht löslich ist. Die Substanz sublimiert zwischen 230 und 270 °C im Vacuum unzersetzt, über 270 °C tritt Zersetzung ein. Die Herstellung der Substanz erfolgt in an sich bekannter Weise durch Umsetzung von Formaldehyd und Ammoniak. Methenamine wird seit Jahrzehnten als Harndesinfektionsmittel in der Human- und Veterinärmedizin bei saurer Reaktion des Harnes eingesetzt, und zwar in Einzeldosen von etwa 0,5 - 1 g und Tagesdosen bis 6 g beim Menschen. In der Technik wird Methenamine zur Herstellung von Kunstharzen, Sprengstoffen, als Vulkanisationsbeschleuniger, zu Gasmaskenfüllungen und in manchen Ländern als Konservierungsmittel für

gewisse Lebensmittel sowie in der chemischen Synthese benutzt. Methenamine gilt als sehr gut verträglich und praktisch nicht toxisch, obgleich es sich im sauren Milieu in toxische Zersetzungsprodukte spaltet. In vitro dissoziiert HMT bei pH 6,0 zu 6% und bei pH 5,0 bereits zu 20% in Formaldehyd und Ammoniumionen. Die Hypothese, daß in vivo HMT überwiegend über die Bildung von Formaldehyd wirkt, konnte vom Bundesgesundheitsamt bei umfangreichen Versuchen im Jahre 1984 nicht bestätigt werden, da sich bei diesen Testen keine Hinweise finden ließen, daß in vivo HMT in ähnlicher Weise wie in vitro gespalten wird. Aufgrund seiner guten Wasserlöslichkeit wird HMT sehr schnell von den Zellen aufgenommen und befindet sich daher bereits nach kurzer Gabe nicht nur im extrazellulären, sondern insbesondere auch im intrazellulären Raum und läßt sich hier noch Stunden nach Gabe einer Einzeldosis nachweisen.

Es ist bekannt, daß im Innern eukaryotische Zellen die endozytischen Vesikeln, Lysosome, Teile des Golgikomplexes und verschiedene sekretorische Vesikeln unterschiedlich saure pH-Werte aufweisen (Madshus in I.H. Biochem. J. 250, 1 (1988));in der eukaryotischen Zelle bestehen verschiedene Systeme für die Regulation des intrazellulären pH-Wertes. Es ist auffallend, daß insbesondere solche Enzyme, die in den DNA-Metabolismus eingreifen, ein Funktionsoptimum bei unterschiedlichen pH-Werten zeigen; beispielsweise benötigen DNA-Polymerasen ein alkalisches Milieu, während einige DNAsen saures Milieu fordern. Die DNAse II hat ihr Funktionsoptimum bei pH-Werten zwischen 4,5 - 5,5 und ihre Aktivität korreliert mit der Zellproliferation (Sierakowska et al in Progress in Nucleic Acid Research and Molecular Biology, W.E. Cohn, Vol. 20, S. 59-130, Academic Press New York (1977)).

Wenn eine Substanz in Zellkulturen zu einer Störung des Wachstums und einer Beeinträchtigung des Zellvolumens führt, wird dies daher in der Regel als zytotoxischer Effekt genereller Art gedeutet, während eine Beeinflussung der Größe des Nucleus als Beeinflussung der DNA-Struktur interpretiert wird.

Es wurde jetzt festgestellt, daß HMT in der Lage ist, in Zellkulturen von Maus-Lyphom L 5178 Y-Zellen die Proliferation der Zellen zu hemmen und Zellvolumen und Zellkerngröße zu vermindern. Dies läßt den Schluß zu, daß HMT in der Lage ist, das Zellwachstum und die DNA Replikation im Nucleus bei Virusinfektionen und in malignen Zellen zu verhindern, was durch die ersten klinischen Versuche bestätigt wird.

Die Erfindung wird im folgenden anhand der Beispiele näher erläutert:

Beispiel 1

Die L 5178 Y Maus-Lyphom Zellen wurden in einem MEM-Spinner Medium mit 10 mmol/l HEPES Puffer, 10% fötalem Kalbserum und 100.000 Einheiten/l Penicillin/Steptomycin (Herstellerin Biocrom, Berlin) kultiviert. Bei Kultivierungsstart lagen 100.000 Zellen/ml Kulturmedium vor, wobei sich 60% der Zellen in der S-Phase befanden. HMT wurde zu den Kulturen in verschiedenen Konzentrationen während einer Zeitspanne von 24 Std. zugegeben. Die Populationen und das Zellvolumen wurden mit einem Zytokombgerät (System Michaelis, Mainz) entsprechend dem Coulter Counter-Prinzip bestimmt. Nach der Zellolyse mit 35 mmol/l Natriumzitrat und 0,5% Triton X 100 (Herstellerin Sigma Chemie, München), RNAse Behandlung und DNAse Färbung mit Propidiumjodid wurde die Kerngröße mit Hilfe eines Impulszytofotometers (FACS analizer) Firma Becton and Dickinson) gemessen.

Die Ergebnisse hinsichtlich Zellwachstum und Zellvolumen sind in den nachfolgenden Tabellen 1 und 2 dargestellt.

Tabelle 1:

| Inhibition des Zellwachstums durch HMT | |
|---|---|
| Konzentration (mg HMT/ml Kulturvolumen) | Zellwachstum (%) |
| Kontrolle | 100 |
| 0,2 | 6 |
| 0,1 | 8 |
| 0,05 | 35 |
| 0,02 | 61 |
| <$1x10^{-3}$ | 100 |

Tabelle 2:

| Änderungen des Zellvolumens durch HMT | |
|---|---|
| Konzentration (mg HMT/ml Kulturvolumen) | Zellvolumen ($\mu m^3$) |
| Kontrolle | 1470,66±390,71 |
| 5 x $10^{-2}$ | 1736,76±438,98 |
| 2 x $10^{-2}$ | 1718,16±487,10 |
| 1 x $10^{-2}$ | 1540,82±401,32 |
| 2 x $10^{-3}$ | 1479,35±387,79 |
| 1 x $10^{-3}$ | 1422,27±398,09 |
| 2 x $10^{-4}$ | 1365,68±371,21 |
| 2 x $10^{-5}$ | 1289,92±350,94 |
| 2 x $10^{-6}$ | 1295,17±379,65 |
| 2 x $10^{-7}$ | 1279,94±388,43 |
| 2 x $10^{-8}$ | 1291,81±410,11 |
| 2 x $10^{-9}$ | 1292,70±398,55 |
| 2 x $10^{-11}$ | 1484,77±412,43 |
| 2 x $10^{-12}$ | 1411,23±395,92 |
| 0,2 | 323,75 |
| 0,1 | 339,06 |
| (Zellfragmente, Mittelwert) | |

Hieraus ergibt sich, daß ab einer Konzentration von 1 x $10^{-3}$/mg/ml HMT das Zellwachstum nicht mehr statistisch signifikant beeinflußt wird, während höhere Konzentrationen an HMT das Zellwachstum inhibieren bzw. zum Zelltod führen. Bei hohen Konzentrationen an HMT lassen sich nur noch Zellfragmente feststellen. Bei Verringerung der Konzentrationen nimmt das Zellvolumen anfangs zu und anschließend wieder ab. Zwischen Konzentrationen von $10^{-4}$ - $10^{-12}$/mg/ml Kulturvolumen korrespondiert das Zellvolumen mit dem Zellvolumen der Kontrollen.

Beispiel 2

Die Zellkulturen wurden wie unter Beispiel 1 beschrieben behandelt. Bei der Bewertung der Nucleusgröße wurde als Kontrolle die Verteilung der Kerngröße bei unbehandelten Zellen zugrunde gelegt. Die Verteilungskurve ergibt sich aus der beigefügten Figur 1.

Bei hohen Konzentrationen von HMT wurden im wesentlichen Zellfragmente festgestellt, während bei verringerten Konzentrationen sich eine konzentrationsabhängige Reduktion der Nucleusgröße im Vergleich zu den Kontrollen zeigte. Im Bereich des normalen Zellvolumens ($10^{-4}$- $10^{-12}$ mg HMT/ml Kulturvolumen) waren aber im Vergleich zu den Kontrollen 25% der Kerne größenreduziert, was mit hoher Wahrscheinlichkeit auf DNA-Vernetzung zurückzuführen ist. Zum Vergleich wurden Versuche mit Cisplatin durchgeführt, von dem bekannt ist, daß es zur DNA-Vernetzung führt. Die Ergebnisse der Versuche sind in der nachfolgenden Tabelle 3 und 4 enthalten.

Tabelle 3:

| Änderungen des Kerns durch HMT | |
|---|---|
| Konzentration (mg HMT/ml Kulturvolumen) | Anzahl der Kerne mit reduzierter Nucleusgröße (%) |
| Kontrolle | - |
| 5 x $10^{-2}$ | 4,87±91,93 |
| 2 x $10^{-2}$ | 6,05 + 2,07 |
| 2 x $10^{-3}$ | 19,62±3,89 |
| 2 x $10^{-4}$ | 13,41±3,71 |
| 2 x $10^{-5}$ | 11,14±3,60 |
| 2 x $10^{-6}$ | 4,50±2,92 |
| 2 x $10^{-7}$ | 1,11±0,58 |
| 2 x $10^{-8}$ | 20,86±4,07 |
| 2 x $10^{-11}$ | 27,17±4,13 |
| 2 x $10^{-12}$ | 25,77±4,29 |
| 0,2 | 81,3 (Kernfragmente) |
| 0,1 | 79,9 (kernfragmente) |
| 0,05 | 25,1 (Kernfragmente) |
| 0,02 | 1,0 (Kernfragmente) |

Tabelle 4:

| Änderungen der Kerngröße durch Cisplatin | |
|---|---|
| Cisplatin (g/ml Kulturvolumen) | Anzahl der Kerne mit reduzierter Nucleusgröße (%) |
| Kontrolle | - |
| 0,5 x $10^{-5}$ | 43,51±5,62 |
| 2,0 x $10^{-6}$ | 18,62±4,08 |
| 1,0 x $10^{-6}$ | 9,95±3,13 |
| 0,5 x $10^{-6}$ | 6,97±2,81 |
| 1,0 x $10^{-7}$ | 4,07±2,24 |

Die in vitro Versuche zeigen deutlich, daß HMT in der Lage ist, bei Konzentrationen, die noch nicht zum Zelltod führen, eine Reduktion des Zellvolumens und der Nucleusgröße zu bewirken. Es wird angenommen, daß HMT in Zellkompartimenten mit sauren pH zu zytotoxischen Folgeprodukten zersetzt wird, die dann ihrerseits, soweit ihre Menge unterhalb der zum Zelltod führenden Menge liegt, zu Veränderungen in der DNA der eukaryotischen Zellen führen, wobei vielleicht der Wirkmechanismus darin besteht, daß die vernetzte DNA auch zu beeinträchtigten Enzymaktivitäten Anlaß gibt, da festgestellt wurde, daß die

Aktivitäten der DNA-Polymerasen und der reversen Transkriptase im Zellkern beeinträchtigt sind. Diese Vermutung wird auch durch die ersten klinischen Anwendungen gestützt.

## Ansprüche

1. Verwendung von Methenamine zur Behandlung von Viruserkrankungen.
2. Verwendung von Methenamine nach Anspruch 1, zur Behandlung von Retroviruserkrankungen.
3. Verwendung von Methenamine zur Behandlung von malignen Neoplasien.
4. Verwendung nach Anspruch 1 bis 3 in Mengen von etwa 2 - 8 g/Tag.

Figur 1

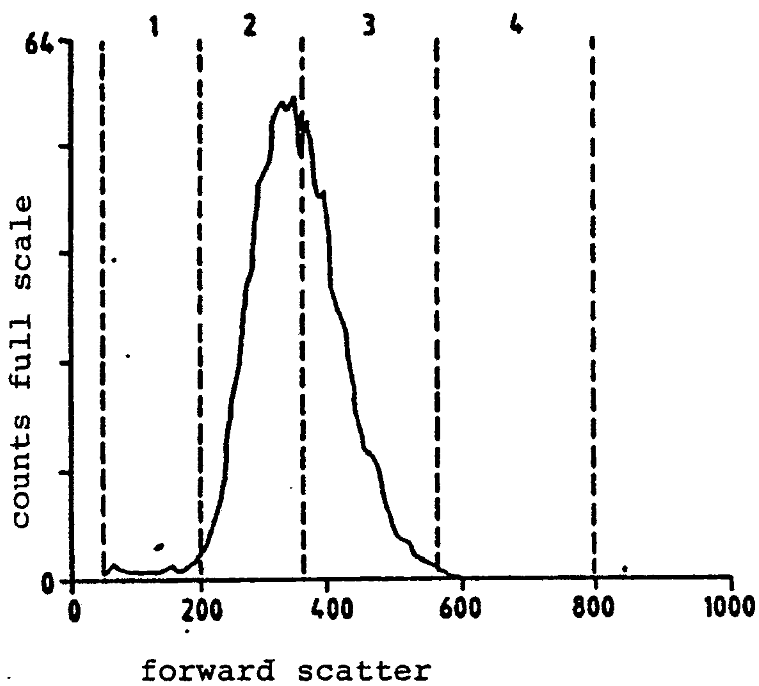


Verteilung der Kerngröße in Kontrollkultur.